(19)

Europäisches
Patentamt
European
Patent Office
Office européen
des brevets

(11) **EP 4 717 759 A2**

(12) **EUROPEAN PATENT APPLICATION**

(43) Date of publication:
**01.04.2026 Bulletin 2026/14**

(21) Application number: **25193711.6**

(22) Date of filing: **04.08.2025**

(51) International Patent Classification (IPC):
*C12M 1/00* (2006.01)    *C12M 1/26* (2006.01)
*C12M 1/42* (2006.01)

(52) Cooperative Patent Classification (CPC):
**C12M 33/08; C12M 23/20; C12M 35/04;
C12M 47/02**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **09.08.2024 JP 2024134387**

(71) Applicant: **Canon Kabushiki Kaisha
Tokyo, 146-8501 (JP)**

(72) Inventors:
• **YAMAMOTO, Yuna**
  **Tokyo (JP)**
• **SURUGA, Eika**
  **Tokyo (JP)**
• **MOCHIZUKI, Shinsuke**
  **Tokyo (JP)**
• **KATO, Mai**
  **Tokyo (JP)**

(74) Representative: **WESER & Kollegen
Patentanwälte PartmbB
Radeckestraße 43
81245 München (DE)**

(54) **CELL CULTURE VESSEL, CELL DETACHMENT METHOD, AND CELL MANUFACTURING METHOD**

(57)     To provide: a cell culture vessel that can achieve both detachability and proliferation ability, that is, can facilitate cell detachment without reducing the proliferation ability of cells; and a cell detachment method using the cell culture vessel (421), provided is a cell detachment method of detaching, through use of a culture vessel (421) having a periodic recess/protrusion pattern on a culture surface, comprising a detachment step of: causing a vibrating unit in which, when an observation image of a cross section perpendicular to the culture surface is obtained, a length Lcp of a contour of the cell on the culture surface side in a measurement region of the observation image and a length Lf of the measurement region in a direction parallel to the culture surface satisfy Lcp>Lf; and transmitting the ultrasonic vibration to the cell, to thereby detach the cell from the culture surface.

FIG. 1

EP 4 717 759 A2

**Description**

TECHNICAL FIELD

The present disclosure relates to a cell culture vessel, a cell detachment method, and a cell manufacturing method.

BACKGROUND

In the fields of cell drugs and regenerative medicine, large amounts of cells are needed, and in particular, there is a demand that adherent cells, which account for many biological tissues, be efficiently and stably supplied.

Adherent cells are manufactured by culturing cells in a culture vessel such as a polystyrene dish and then detaching and collecting the cells from the culture vessel. In order to efficiently and stably supply adherent cells, there is a demand for a method that facilitates detachment of adherent cells.

In Japanese Patent Laid-Open No. 2005-168494 and Japanese Patent Laid-Open No. 2019-37151, there are proposals for a method that facilitates the detachment by forming a group of protrusions on a culture surface and bringing the cells into point contact or the like with the culture surface to reduce an adhesion area and thereby weaken adhesion strength.

The related art has room for improvement in terms of detachability and proliferation ability of cells.

SUMMARY

The present disclosure provides methods for cell detachment.

The present disclosure in its first aspect provides a cell detachment method as specified in claim 1. Optional features are specified in claims 2 to 7.

The present disclosure in its second aspect provides a cell detachment method as specified in claim 8. Optional features are specified in claims 9 and 10.

The present disclosure in its third aspect provides a cell manufacturing method as specified in claim 11.

The present disclosure in its fourth aspect provides a cell manufacturing method as specified in claim 12.

The present disclosure in its fifth aspect provides A cell culture vessel as specified in claim 13. Optional features are specified in claims 14 to 16.

According to the present disclosure, it is possible to provide: a cell culture vessel that can achieve both detachability and proliferation ability; a cell detachment method that enables detachment while maintaining proliferation ability; and a cell manufacturing method that achieves both proliferation ability and detachability.

Features of the present disclosure will become apparent from the following description of embodiments with reference to the attached drawings. The following description of embodiments is described by way of example.

BRIEF DESCRIPTION OF THE DRAWINGS

Fig. 1 shows an example of a cross-sectional observation image of a recess/protrusion pattern and a cell.
Fig. 2A is illustrations of an example of the recess/protrusion pattern having protruding portions.
Fig. 2B is illustrations of an example of the recess/protrusion pattern having recessed portions.
Fig. 3A is an illustration of an example of the recess/protrusion pattern.
Fig. 3B is an illustration of an example in which the recess/protrusion pattern is formed of cylindrical protruding portions.
Fig. 3C is an illustration of an example in which the recess/protrusion pattern is formed of cylindrical recessed portions.
Fig. 3D is an illustration of an example in which the recess/protrusion pattern is formed of protruding portions each having a truncated conical shape.
Fig. 3E is an illustration of an example in which the recess/protrusion pattern is formed of recessed portions each having a truncated conical shape.
Fig. 4 a flowchart of a cell detachment method and a cell manufacturing method.
Fig. 5 is a schematic diagram of a mechanism for applying ultrasonic vibration.
Fig. 6A is views for illustrating evaluation of an adhering state of a cell from a cross-sectional observation image.
Fig. 6B is views for illustrating evaluation of an adhering state of a cell from a cross-sectional observation image.
Fig. 7A is a schematic view of a detachment mechanism based on a jet.
Fig. 7B is a schematic view of the detachment mechanism based on a jet.

DESCRIPTION OF THE EMBODIMENTS

It has been found that weakening adhesion strength of cells as in the related art may reduce proliferation of cells when dealing with such adherent cells as to exert the proliferation ability through strong adhesion, and may lead to a failure in obtaining a sufficient number of cells.

The present disclosure is directed to: a cell culture vessel that can achieve both detachability and proliferation ability, that is, can facilitate cell detachment without reducing the proliferation of cells; and a cell detachment method using the cell culture vessel.

The inventors have found a method for detachment when an adhesion area is large in contrast to the method described in the related art in which a contact area is reduced to facilitate the detachment. That is, the inventors have found that it is possible to achieve both the detachability and the proliferation ability of cells when vibration in an ultrasonic band is applied under a state in which a cell adhesion area per unit region of a culture surface is large.

The inventors consider that a reason why a culture vessel, a cell detachment method, and a cell manufacturing method according to the present disclosure can achieve both the detachability and the proliferation ability of cells is as follows.

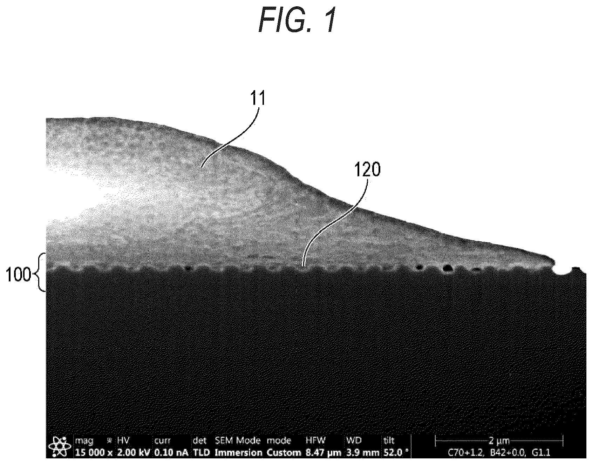

When cells are cultured in the culture vessel according to the present disclosure, a cell 11 even enters gaps 120 of a recess/protrusion pattern 100 as shown in Fig. 1 and adheres thereto, thereby enabling culture of the cell without reducing the proliferation ability. Then, in the present disclosure, the cell 11 is detached by applying vibration in an ultrasonic band instead of by a detachment method utilizing a flow such as a jet. When the vibration in an ultrasonic band is applied under a state in which an adhesion area of the cell 11 per unit region of the culture surface is large, the cell receives ultrasonic vibration from various angles, and energy required for the detachment can be efficiently applied to the adhesion surface.

This enables the cell 11 to be detached more efficiently than by applying the vibration in an ultrasonic band to a normal culture surface that does not have the recess/protrusion pattern 100. Accordingly, it is possible to improve the detachability without reducing the adhesion area of the cell 11, and it is considered that it has been possible to successfully achieve both the detachability and the proliferation ability of the cell 11, which was difficult in the related art.

Now, embodiments regarding a cell culture vessel, a cell detachment method, and a cell manufacturing method according to first and second embodiments of the present disclosure are described. The embodiments described below do not limit the present disclosure set forth in the appended claims.

<First Embodiment>

As the first embodiment, the present disclosure provides a culture vessel comprising a periodic recess/protrusion pattern on a culture surface, wherein a pitch P of the periodic recess/protrusion pattern on the culture surface satisfies 100 nm$\leq$P$\leq$2,000 nm, and wherein the periodic recess/protrusion pattern includes at least any one of the following (A) or (B): (A) a plurality of protruding portions which protrude from a reference plane of the periodic recess/protrusion pattern, and are periodically arranged, where the plurality of protruding portions are arranged at a height H and an interval I, and 20 nm$\leq$H$\leq$400 nm and H/I$\leq$1.1 are satisfied; and (B) a plurality of recessed portions which are recessed from the reference plane of the periodic recess/protrusion pattern, and are periodically arranged, where the plurality of recessed portions have a depth D and a hole diameter S, and 20 nm$\leq$D$\leq$400 nm and D/S$\leq$1.1 are satisfied.

(Culture Vessel)

The culture vessel according to this embodiment is not particularly limited as long as the culture vessel includes a cell-adhesive culture surface, and examples thereof include a flask, a flask for tissue culture, a dish, a Petri dish, a dish for tissue culture, a multidish, a microplate, a multiwell plate, a multiplate, a Petri plate, a culture bag, and a bottle. Further, the culture surface refers to a surface which is located inside the culture vessel among surfaces forming the culture vessel and to/on which cells can mainly adhere and grow.

A material for the culture vessel according to this embodiment is only required to be a material that is chemically stable and capable of culturing desired cells, and examples thereof include polyethylene, polypropylene, polycarbonate, polystyrene, polyvinyl chloride, nylon, polyurethane, polyurea, polylactic acid, polyglycolic acid, polyvinyl alcohol, polyvinyl acetate, poly(meth)acrylic acid, poly(meth)acrylic acid derivatives, polyacrylonitrile, poly(meth)acrylamide, poly(meth)acrylamide derivatives, polysulfone, cellulose, cellulose derivatives, polysilicone, polymethylpentene, glass, and metals. In some embodiments, the culture vessel can be polystyrene.

(Recess/protrusion Pattern)

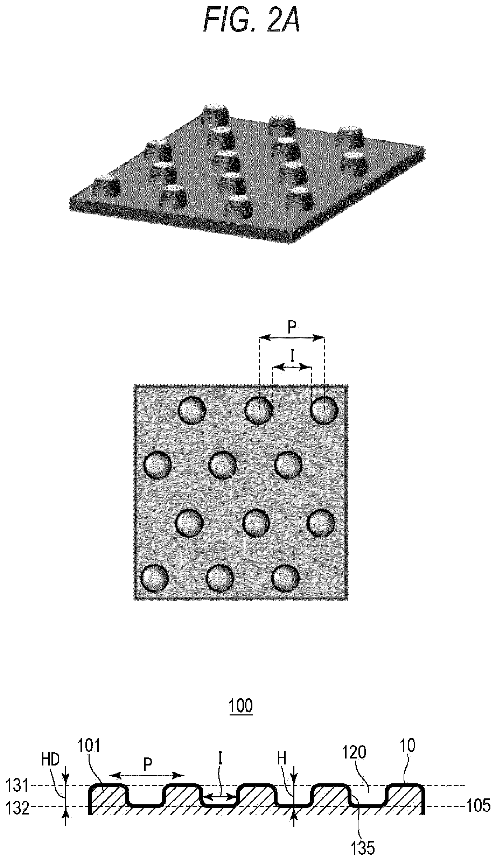

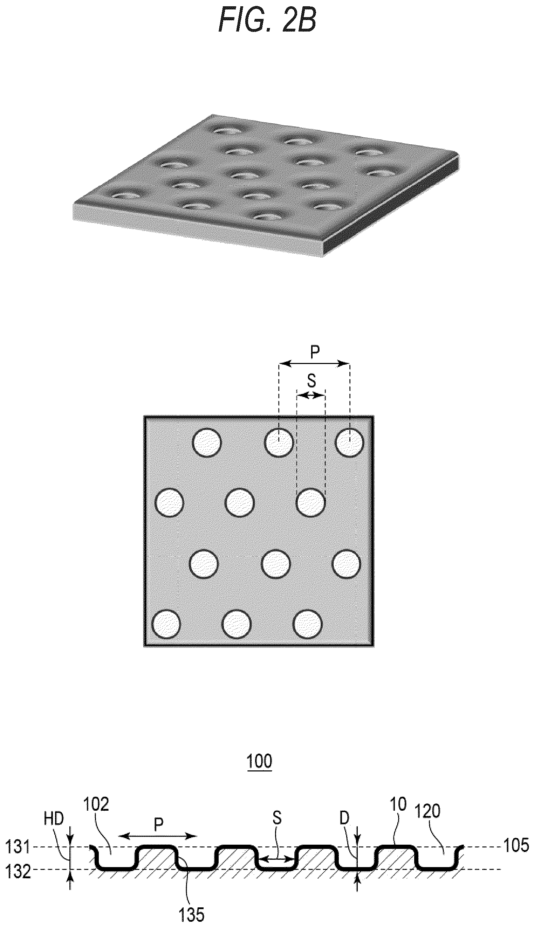

Specific examples of a recess/protrusion pattern in this embodiment are illustrated in Fig. 2A and Fig. 2B. In both figures, a perspective view of the recess/protrusion pattern is illustrated at the top, a top view is illustrated in the middle, and a cross-sectional view is illustrated at the bottom. A culture surface 10 indicated by the thick line in Fig. 2A and Fig. 2B has the recess/protrusion pattern 100. The culture surface 10 having the recess/protrusion pattern 100 refers to the culture surface 10 being provided with a periodic recess/protrusion pattern (design) with a height difference (height difference HD is described later) of at least 10 nm. The periodic recess/protrusion pattern 100 refers to a pattern in which a recess and a protrusion are repeated at approximately regular intervals. The recess and the protrusion have relative meanings. A recessed portion 102 and a protruding portion 101 as used herein do not have relative meanings, but are based on a reference plane 105. The protruding portion 101 is set as a portion protruding from the reference plane 105 of the culture surface 10, and the recessed portion 102 is set as a portion recessed from the reference plane 105 of the culture surface 10. The reference plane 105 is provided for convenience, and can be freely determined. Further, the recess/protrusion pattern being periodic enables cells to grow uniformly and to be detached uniformly by application of the vibration in an ultrasonic band.

The illustrations of Fig. 2A and Fig. 2B include a highest surface 131 of the culture surface 10, a lowest surface 132 of the culture surface 10, and a side surface 135 of each recessed/protruding portion (recessed portion 102 and/or protruding portion 101). In Fig. 2A, the lowest surface 132 is flush with the reference plane 105, and in Fig. 2B, the highest surface 131 is flush with the reference plane 105.

The recess/protrusion pattern 100 is formed by the protruding portions 101 that are periodically arranged and/or the recessed portions 102 that are periodically arranged. Fig. 2A is an illustration of an example in which the recess/protrusion pattern 100 is formed by the protruding portions 101, and Fig. 2B is an illustration of an example in which the recess/protrusion pattern 100 is formed by the recessed portions 102.

The gap 120 or simply "recess" as used herein refers to a relative recess regardless of the reference plane 105. For example, as illustrated in Fig. 2A, a space interposed between a plurality of protruding portions 101 is the gap (recess) 120, and as illustrated in Fig. 2B, the recessed portion 102 is also the gap 120. The same holds true for the protrusion, and both a portion interposed between a plurality of recessed portions 102 and the protruding portion 101 can be said to be protrusions.

In the example illustrated in Fig. 2A, the protruding portions 101 protrude from the reference plane 105 of the recess/protrusion pattern 100, and are periodically arranged at a height H and an interval I, where 100 nm≤I≤2,000 nm, 20 nm≤H≤400 nm, and H/I≤1.1 are satisfied.

In the example illustrated in Fig. 2B, the recessed portions 102 are recessed from the reference plane 105 of the recess/protrusion pattern 100, and are periodically arranged with a depth D and a hole diameter S, where 100 nm≤S≤2,000 nm, 20 nm≤D≤400 nm, and D/S≤1.1 are satisfied.

A pitch P refers to a distance from a center of a recess to a center of an adjacent recess or from a center of a protrusion to a center of an adjacent protrusion in a top view of the recess/protrusion pattern 100.

The pitch P can satisfy 100 nm≤P≤2,000 nm. When the pitch P falls within this range, the surface area of the culture surface 10 is increased, and a cell (not shown in Fig. 2A and Fig. 2B) can easily enter the gaps 120, thereby increasing the cell adhesion area per unit region of the culture surface. This enables ultrasonic vibration to act on the cell from various directions at the time of detachment using the application of vibration in an ultrasonic band, thereby being able to improve the detachability. In addition, even at the time of culture, when the adhesion area is increased, it becomes difficult for an adhesion force to decrease, and hence high proliferation ability is obtained. Thus, it is possible to achieve both the detachability and the proliferation ability.

In contrast, when the pitch P is less than 100 nm, the opening portion of the gap 120 is small, and it becomes difficult for a cell to enter the gap 120, resulting in a small adhesion area and thereby making it difficult to increase the proliferation ability. Further, when the pitch P is more than 2,000 nm, the interval I between the protruding portions 101 or the hole diameter S of the recessed portion 102 becomes too wide, thereby causing a large part of the cell to adhere to one recessed/protruding portion, and it becomes difficult to increase the cell adhesion area per unit region of the culture surface, thereby making it difficult to obtain a detachment effect obtained by applying ultrasonic vibration when the cell adhesion area is large.

Further, the pitch P can fall within a range that allows a cell to easily enter the gaps 120 and to be sufficiently small relative to a size of a cell to be used, and the pitch P satisfying 100 nm≤P≤1,000 nm is better.

The recess/protrusion pattern height difference HD refers to a height difference between the highest surface 131 and the lowest surface 132 of the recess/protrusion pattern 100. The height H of the protruding portion 101 can be the recess/protrusion pattern height difference HD when the recess/protrusion pattern 100 is formed of the protruding portions 101, the depth D of the recessed portion 102 can be the recess/protrusion pattern height difference HD when the recess/protrusion pattern 100 is formed of the recessed portions 102, and (height H)+(depth D) can be the recess/protrusion pattern height difference HD when both the protruding portions 101 and the recessed portions 102 are present. The recess/protrusion pattern height difference HD can satisfy 20 nm≤HD≤400 nm. When the recess/protrusion pattern height difference HD is within this range, the surface area of the culture surface 10 is increased, and a cell can reach the lowest surface 132 of the recess/protrusion pattern 100 and easily adhere thereto, thereby increasing the cell adhesion area per unit region of the culture surface. This enables vibration in an ultrasonic band to act on the cell from various directions at the time of detachment using the application of the vibration in an ultrasonic band, thereby being able to improve the detachability.

In addition, it becomes difficult for the adhesion force to decrease at the time of culture, and hence high proliferation is obtained. Thus, it is possible to achieve both the detachability and the proliferation ability.

In contrast, when the recess/protrusion pattern height difference HD is less than 20 nm, the surface area of the culture surface 10 is not increased, and the cell adhesion area per unit region of the culture surface is not increased, thereby making it difficult to obtain the detachment effect obtained by applying ultrasonic vibration when the cell adhesion area is large. When the recess/protrusion pattern height difference HD is more than 400 nm, it becomes difficult for a cell to adhere to an entire structure forming the recess/protrusion pattern, and hence the adhesion area is reduced, thereby making it difficult to increase the proliferation ability.

The recess/protrusion pattern height difference HD falling within a range of from 20 nm to 400 nm inclusive facilitates improvement in initial adhesion of a harvested cell. In a related-art cell detachment method, the detachment is performed through use of an enzyme such as trypsin, and hence membrane protein of the cell is easily reduced, thereby easily lowering the initial adhesion of the harvested cell. In contrast, in the present disclosure, it is possible to easily detach a cell by applying vibration in an ultrasonic band to the cell under a state in which the cell adhesion area per unit region of the culture surface is increased by the culture surface 10 having the recess/protrusion pattern 100, and hence a cell containing a large amount of membrane protein can be harvested. Thus, it is presumed that the cell detached by the detachment method according to the present disclosure has a higher initial adhesion than that of the cell detached by the related-art method. For example, through use of cells detached by the detachment method according to the present disclosure, it is possible to increase culture efficiency, which is particularly effective in performing mass culture.

The recess/protrusion pattern height difference HD can be 20% or less of a thickness of a cell at the time of adhesion, and from this viewpoint, can satisfy 50 nm$\leq$HD$\leq$300 nm. 100 nm$\leq$HD$\leq$300 nm is better.

In this embodiment, it is suitable that a ratio H/I of the height H of the protruding portion 101 to the interval I thereof and a ratio D/S of the depth D of the recessed portion 102 to the hole diameter S thereof be both 1.1 or less. The interval I is a length obtained by subtracting distances including the protruding portions 101 from the pitch P in the top view of a recess/protrusion pattern when the recess/protrusion pattern is formed by the protruding portions. For example, when the protruding portion 101 has a cylindrical shape, the interval I is a length obtained by subtracting a diameter of the cylinder from the pitch P. When the protruding portion 101 has an elliptic cylindrical shape, the interval I is set as a length obtained by subtracting a minor axis length of the elliptic cylinder (length of the shorter one of the orthogonal axes of the ellipse) from the pitch P. The hole diameter S is a diameter of the recessed portion when the recessed portion has a cylindrical shape. When the recessed portion has an elliptic cylindrical shape, the hole diameter S is set as the minor axis length. When the side surface 135 of each recessed/protruding portion is not perpendicular to the culture surface 10 (for example, when the recessed/protruding portion has a truncated conical shape), the hole diameter of the recessed/protruding portion is set as a hole diameter of the recessed/protruding portion obtained at a point located at half the depth D of the recessed portion or at a point located at half the height H of the protruding portion. The ratios H/I and D/S being 1.1 or less allow a cell to easily enter the recessed portions and increase the adhesion area, and hence it is possible to achieve both the detachability and the proliferation ability.

The shape of the recessed/protruding portion that forms the recess/protrusion pattern 100 is not particularly limited. The recess/protrusion pattern 100 can have a pillar structure, a moth-eye structure, or a hole structure, and the recessed/protruding portion may have a columnar shape, a conical shape, or a frustum shape.

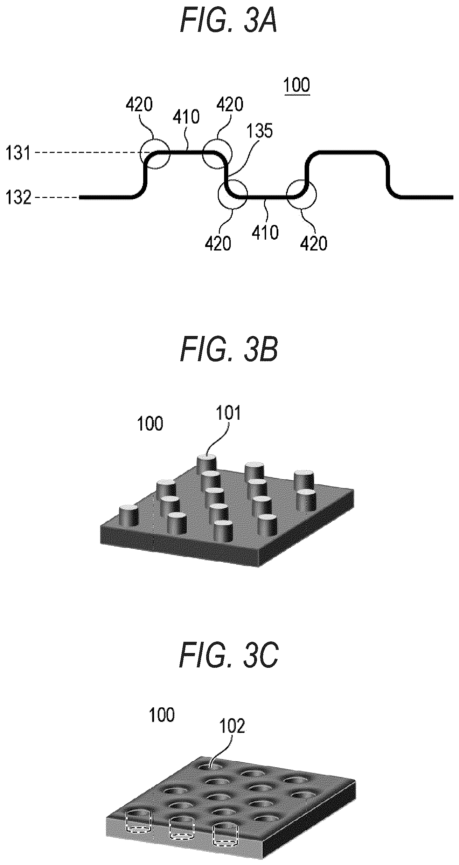

As exemplified in Fig. 3A, the highest surface 131 of the recess/protrusion pattern 100 can have a flat surface portion 410. Meanwhile, the highest surface 131 of the recess/protrusion pattern 100 and the side surface 135 of each recessed/protruding portion can be contiguous to each other through a curved surface portion 420. In addition, the lowest surface 132 of the recess/protrusion pattern 100 can have a flat surface portion 410. Meanwhile, the lowest surface 132 of the recess/protrusion pattern 100 and the side surface 135 of each recessed/protruding portion can be contiguous to each other through a curved surface portion 420.

The recess/protrusion pattern 100 having the flat surface portions 410 facilitates the adhesion of a cell (not shown in Fig. 3A to Fig. 3E), and the recess/protrusion pattern 100 having the curved surface portions 420 makes it easy for the cell to follow the recess/protrusion pattern 100. As a result, the cell adhesion area is increased, and both the detachability and the proliferation ability are easily achieved.

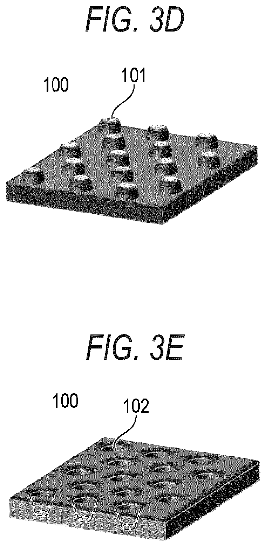

Further, as illustrated in Fig. 3B to Fig. 3E, in particular, the shape of the recessed/protruding portion that forms the recess/protrusion pattern 100 can have a cylindrical shape or a truncated conical shape. Fig. 3B is an illustration of an example in which the recess/protrusion pattern 100 is formed of cylindrical protruding portions 101, Fig. 3C is an illustration of an example in which the recess/protrusion pattern 100 is formed of cylindrical recessed portions 102, Fig. 3D is an illustration of an example in which the recess/protrusion pattern 100 is formed of protruding portions 101 each having a truncated conical shape, and Fig. 3E is an illustration of an example in which the recess/protrusion pattern 100 is formed of recessed portions 102 each having a truncated conical shape. When the side surface 135 of each recessed/protruding portion is formed of a curved surface or has a curved surface, a cell easily follows the recess/protrusion pattern 100. As a result, the cell adhesion area is increased, and both the detachability and the proliferation ability are easily achieved.

From the viewpoints of strength and transmission of ultrasonic vibration, the recess/protrusion pattern 100 can be formed integrally with the culture vessel, and from the viewpoint of processing, the culture surface having the recess/protrusion pattern 100 can be made of resin. However, the material therefor is not particularly limited as long as the material allows cells to adhere thereto.

A method of forming a recess/protrusion pattern on a culture surface is not particularly limited, but from the viewpoint of mass productivity, it can employ a shape transfer method using a mold on which a recess/protrusion pattern is formed such as a nanoimprinting method or an injection molding method. As a method of forming a recess/protrusion pattern on a mold or a culture surface, a micromachining method such as a photolithography method, an electron beam direct drawing method, a particle beam processing method, or a scanning probe processing method is used. The culture surface comprising the recess/protrusion pattern may be subjected to surface treatment suited for the culture of cells.

(Cells)

The cells to be used in the present disclosure are not particularly limited as long as the cells can adhere to, and be cultured on, the culture surface of a culture vessel *in vitro.* Examples of the cells include various cultured cell lines, such as Chinese hamster ovary-derived CHO cells, mouse connective tissue L929 cells, mouse skeletal muscle myoblasts (C2C12 cells), human fetal lung-derived normal diploid fibroblasts (TIG-3 cells), human embryonic kidney-derived cells (HEK293 cells), human alveolar basal epithelial adenocarcinoma-derived A549 cells, mouse macrophage-like cells (RAW264.7), and human cervical cancer-derived HeLa cells. In addition thereto, examples of the cells include: epithelial cells and endothelial cells making up various tissues and organs in living bodies, skeletal muscle cells, smooth muscle cells, and cardiac muscle cells exhibiting contractility, neuronal cells and glial cells making up the nervous system, fibroblasts, and hepatic parenchymal cells, hepatic nonparenchymal cells, and adipocytes involved in metabolisms in living bodies; as cells having differentiation potency, various stem cells, such as induced pluripotent stem (iPS) cells, embryonic stem (ES) cells, embryonic germ (EG) cells, embryonal carcinoma (EC) cells, mesenchymal stem cells, hepatic stem cells, pancreatic stem cells, skin stem cells, muscle stem cells, and germline stem cells, or progenitor cells of various tissues; and cells obtained by inducing differentiation of those cells.

(Cell Detachment Method and Cell Culture Method)

The first embodiment of the present disclosure further provides the following cell detachment method.

There is provided a cell detachment method comprising use of a culture vessel having a periodic recess/protrusion pattern on a culture surface, a cell adhering to the culture surface from the culture surface, the cell detachment method comprising a detachment step of: causing a vibrating unit arranged and configured to apply vibration to the culture vessel to generate ultrasonic vibration; and transmitting the ultrasonic vibration to the cell, to thereby detach the cell from the culture surface, wherein a pitch P of the periodic recess/protrusion pattern on the culture surface satisfies $100 \text{ nm} \leq P \leq 2,000 \text{ nm}$, and wherein the culture surface includes at least any one of the following (A) or (B): (A) a plurality of protruding portions which protrude from a reference plane of the periodic recess/protrusion pattern, and are periodically arranged, where the plurality of protruding portions are arranged at a height H and an interval I, and $20 \text{ nm} \leq H \leq 400 \text{ nm}$ and $H/I \leq 1.1$ are satisfied; and (B) a plurality of recessed portions which are recessed from the reference plane of the periodic recess/protrusion pattern, and are periodically arranged, where the plurality of recessed portions have a depth D and a hole diameter S, and $20 \text{ nm} \leq D \leq 400 \text{ nm}$ and $D/S \leq 1.1$ are satisfied.

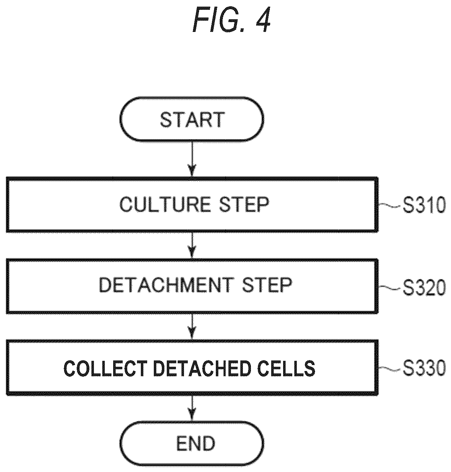

The cell detachment method according to this embodiment is illustrated in Fig. 4, includes a detachment step S320, and may further include a culture step S310 of culturing cells and a collection step S330 of collecting the detached cells. However, the culture step S310 and the collection step S330 are not essential.

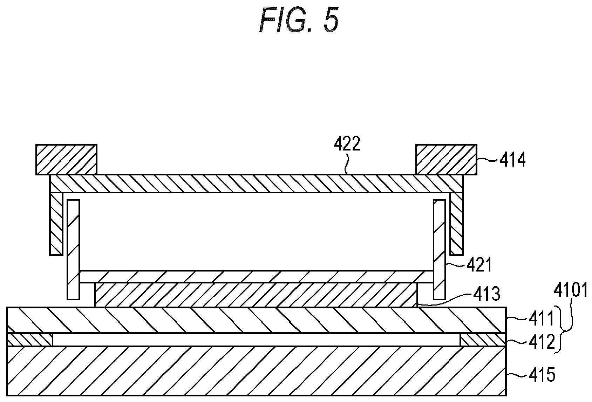

A specific example thereof is described with reference to Fig. 4 and Fig. 5.

First, as the culture step, cells are cultured in a culture vessel having a culture surface having the recess/protrusion pattern (Step S310).

Subsequently, the detachment step of applying vibration in an ultrasonic band is started (Step S320). For example, as illustrated in Fig. 5, a mechanism for applying the vibration in an ultrasonic band to the culture vessel can be used for the application of the vibration in an ultrasonic band. In Fig. 5, the mechanism for applying the vibration in an ultrasonic band includes a vibrating body (ring device) 4101, a vibration transmitting member 413, and a cover 415, and the vibrating body (ring device) 4101 includes a diaphragm 411 and a piezoelectric body 412. The culture vessel 421 is placed on the vibration transmitting member 413. In the example illustrated in Fig. 5, the culture vessel 421 is covered with a culture vessel upper lid 422, and a weight 414 is placed on the culture vessel upper lid 422. In this manner, when the culture vessel 421 is covered with the culture vessel upper lid 422 and the weight 414 is further placed thereon, it may be possible to efficiently apply the vibration in an ultrasonic band. Finally, the detached cells are collected (Step S330).

6

The first embodiment of the present disclosure further provides the following cell manufacturing method.

There is provided a cell manufacturing method comprising: a culture step of culturing a cell in a culture vessel having a periodic recess/protrusion pattern on a culture surface; and a detachment step of detaching the cell adhering to the culture surface from the culture surface, wherein the detachment step includes: causing a vibrating unit arranged and configured to apply vibration to the culture vessel to generate ultrasonic vibration; and transmitting the ultrasonic vibration to the cell, to thereby detach the cell from the culture surface, wherein a pitch P of the periodic recess/protrusion pattern on the culture surface satisfies $100\ nm \leq P \leq 2{,}000\ nm$, and wherein the periodic recess/protrusion pattern includes at least any one of the following (A) or (B): (A) a plurality of protruding portions which protrude from a reference plane of the periodic recess/protrusion pattern, and are periodically arranged, where the plurality of protruding portions are arranged at a height H and an interval I, and $20\ nm \leq H \leq 400\ nm$ and $H/I \leq 1.1$ are satisfied; and (B) a plurality of recessed portions which are recessed from the reference plane of the periodic recess/protrusion pattern, and are periodically arranged, where the plurality of recessed portions have a depth D and a hole diameter S, and $20\ nm \leq D \leq 400\ nm$ and $D/S \leq 1.1$ are satisfied.

The cell manufacturing method according to this embodiment is also illustrated in Fig. 4, and includes the culture step S310 and the detachment step S320. The collection step S330 of collecting the detached cells may be further included, but the collection step S330 is not essential.

Each of the steps is described below.

(Culture Step)

As the culture step, a publicly-known cell culture method can be used. Details thereof are described below.

(Cell Culture Method)

Culture conditions for the cells may be appropriately selected in accordance with the cells to be cultured. In general, an appropriate medium is added into the culture vessel, and the cells are seeded therein at from about $1.0 \times 10^1$ cells/cm$^2$ to about $5.0 \times 10^4$ cells/cm$^2$ and are cultured under an environment at 37°C and a $CO_2$ concentration of 5%. At this time, the cells can be cultured until a cell coverage area ratio of the culture surface of from about 70% to about 80%, that is, a so-called subconfluent state is achieved.

(Medium)

The kind of the medium is not particularly limited, and examples thereof include Dulbecco's Modified Eagles's Medium (DMEM), Ham's Nutrient Mixture F12, DMEM/F12 medium, McCoy's 5A medium, Eagles's Minimum Essential Medium (EMEM), alpha Modified Eagles's Minimum Essential Medium ($\alpha$MEM), Minimum Essential Medium, RPMI 1640 medium, Iscove's Modified Dulbecco's Medium (IMDM), MCDB 131 medium, Williams' Medium E, IPL 41 medium, Fischer's medium, StemSpan H3000 (manufactured by STEMCELL Technologies), StemSpan SFEM (manufactured by STEMCELL Technologies), Stemline II (manufactured by Sigma-Aldrich), Endothelial Cell Growth Medium 2 Kit (manufactured by PromoCell), Mesenchymal Stem Cell Growth Medium 2 (manufactured by PromoCell), MSCGM Bullet Kit (manufactured by Lonza), mTeSR1 or mTeSR2 medium (manufactured by STEMCELL Technologies), REPRO FF or REPRO FF2 (manufactured by REPROCELL), NutriStem medium (manufactured by Biological Industries), and MF-Medium mesenchymal stem cell growth medium (manufactured by TOYOBO Co., Ltd.).

Of those, a medium suited for the culture of each type of cells is suitable to be used.

(Serum)

Serum may be added to the above-mentioned medium. Examples of the serum to be used include fetal bovine serum (FBS), calf serum, adult bovine serum, horse serum, sheep serum, goat serum, porcine serum, chicken serum, rabbit serum, and human serum, and FBS is often commonly used because of its ease of availability. In addition, a serum-free medium being free of any untreated or unpurified serum and containing a purified blood-derived component or animal tissue-derived component (for example, a growth factor) may be used.

(Antibiotic)

An antibiotic may be added to the above-mentioned medium. Examples of the antibiotic to be added to the medium include penicillin, streptomycin, ampicillin, carbenicillin, tetracycline, bleomycin, actinomycin, kanamycin, actinomycin D, and amphotericin B.

(Detachment Step)

The detachment step includes a vibration application step, and applies vibration in an ultrasonic band to cells, to thereby detach the cells. Details thereof are described below.

(Application of Ultrasonic Vibration)

A vibrating unit arranged so as to be able to apply vibration to the culture vessel is caused to generate ultrasonic vibration, to thereby apply ultrasonic vibration to the culture vessel, and the ultrasonic vibration is transmitted to the cells. The ultrasonic vibration refers to vibration in an ultrasonic band which has a frequency of from 10 kHz to 1 MHz inclusive. There are no particular limitations on the vibrating unit as long as the vibrating unit can apply ultrasonic vibration to the culture vessel and transmit the ultrasonic vibration to the cells. For example, a vibrating body such as an ultrasonic oscillator can be used.

Further, specific examples of the vibrating body include a ring device obtained by bonding a ring-shaped piezoelectric body to a glass plate or a metal plate, and a Langevin-type transducer (Langevin device).

The ultrasonic vibration can be applied by bringing the vibrating body into direct contact with an outer surface of the culture vessel, or can be applied through intermediation of the vibration transmitting member placed between the vibrating body and the outer surface of the culture vessel. Further, the ultrasonic vibration is thus transmitted to the cells to be detached through intermediation of the culture vessel. In particular, high detachability is easily obtained by transmitting the ultrasonic vibration to the cells through intermediation of the culture surface having the recess/protrusion pattern, and hence it is suitable to apply vibration from a back side of the culture surface among the outer surfaces of the culture vessel. In addition, the vibration transmitting member may be made of any material that can transmit the ultrasonic vibration to the culture vessel, and examples thereof include liquid materials such as water and glycerin and solid materials such as rubber and gel.

The maximum amplitude of the ultrasonic vibration on the vibrating surface is not particularly limited, but from the viewpoint of maintaining the cell viability, is desired to be 10 $\mu$m or less. This range can facilitate the detachment of the cells while suppressing the influence of heat generated by the vibrating body. The above-mentioned range is also suitable because, when the amplitude is set to 10 $\mu$m or less, cavitation is less likely to occur at the time of application of the ultrasonic vibration, and the cell viability is less likely to be lowered.

As a vibration pattern of the ultrasonic vibration, it is possible to use various vibration modes depending on the vibrating body to be used as the vibrating unit and the type of ultrasonic device.

Further, depending on the type of vibrating body or the driving method therefor, heat may be generated during driving. This tendency tends to intensify by continuing driving at a resonance frequency, which depends on the applied voltage, the frequency band that is used, and the apparatus environment. In view of this, it is possible to control a heat generation amount by performing, alone or in combination, repetition of driving and pausing at regular intervals (burst driving), repeated and continuous changing of a driving frequency within a fixed frequency range (sweep driving), or the like. In addition, in order to suppress the heat generation, a mechanism for cooling can be provided in the periphery of the vibrating unit to control the vibrating body directly or indirectly through an environmental temperature. Further, the resonance frequency may change due to the heat generation from the vibrating body during driving, and in that case, a publicly-known method such as resonance frequency tracking control based on current detection can be performed.

The environmental temperature to be used when the ultrasonic vibration is applied is not particularly limited, and can be set appropriately depending on the type of cells to be used. From the viewpoint of maintaining the cell viability, the environmental temperature can be 20°C or more and 40°C or less. For example, in a case of mammalian cells, the environmental temperature can be 30.0°C or more and 37.5°C or less. That is because, with this range, the environmental temperature is close to the temperature at the time of culture, and hence the influence of temperature change on the cells can be reduced, with the result that the cell viability can be kept high. The application of the ultrasonic vibration may also be performed under an environment at a $CO_2$ concentration of 5% in the same manner as in the cell culture. The environmental conditions to be used when the ultrasonic vibration is applied can be set appropriately depending on the type and characteristics of cells, and the method of using the cells after the detachment.

In addition to the vibration application step, the detachment step may include a liquid replacement step of replacing the medium in the culture vessel by a buffer or the like immediately before the vibration application step for the purpose of promoting cell detachment. Alternatively, the liquid replacement step may be performed before the detachment step.

Any buffer may be used without any limitation as long as a neutral region can be kept. Examples thereof include a Tris buffer such as a Tris-HCl buffer, a phosphate buffer, a HEPES buffer, a citrate-phosphate buffer, a glycylglycine-sodium hydroxide buffer, a Britton-Robinson buffer, and a GTA buffer. Of those, a phosphate buffer that is close to an *in vivo* environment can be used, and phosphate buffered saline (PBS) obtained by adjusting the phosphate buffer so as to be isotonic to an intracellular fluid is more suitably used. When PBS is used, it is suitable to use PBS(-) free of divalent cations

such as $Ca^{2+}$ and $Mg^{2+}$.

Further, the detachment step may also include an external stimulus application step in which an external stimulus that generates convection or impact is applied to the culture surface during the detachment step for the purpose of promoting cell detachment. As a method of generating convection, there is pipetting, a method using a pump, an agitator, or a shaker, or the like, and as a method of generating impact, there is tapping on a wall surface or a bottom surface.

<Second Embodiment>

As the second embodiment of the present disclosure, the following cell detachment method is provided.

There is provided a cell detachment method comprising use of a culture vessel having a periodic recess/protrusion pattern on a culture surface, a cell adhering to the culture surface from the culture surface, the cell detachment method comprising a detachment step of: causing a vibrating unit arranged and configured to apply vibration to the culture vessel to generate ultrasonic vibration under a state in which, when an observation image of a cross section perpendicular to the culture surface is obtained, a length Lcp of a contour of the cell on the culture surface side in a measurement region of the observation image and a length Lf of the measurement region in a direction parallel to the culture surface satisfy Lcp>Lf; and transmitting the ultrasonic vibration to the cell, to thereby detach the cell from the culture surface.

In addition, as the second embodiment of the present disclosure, the following cell manufacturing method is provided.

There is provided a cell manufacturing method comprising: a culture step of culturing a cell in a culture vessel having a periodic recess/protrusion pattern on a culture surface; and a detachment step of detaching the cell adhering to the culture surface from the culture surface, wherein the detachment step includes: causing a vibrating unit arranged and configured to apply vibration to the culture vessel to generate ultrasonic vibration under a state in which, when an observation image of a cross section perpendicular to the culture surface is obtained, a length Lcp of a contour of the cell on the culture surface side in a measurement region of the observation image and a length Lf of the measurement region in a direction parallel to the culture surface satisfy Lcp>Lf; and transmitting the ultrasonic vibration to the cell, to thereby detach the cell from the culture surface.

(Recess/protrusion Pattern)

In regard to the recess/protrusion pattern, the description in the first embodiment can be referred to. In this embodiment, the pitch P of the recessed/protruding portions can satisfy 100 nm≤P≤2,000 nm. When the pitch P falls within this range, the surface area of the culture surface 10 is increased, and a cell can easily enter the gaps 120, thereby increasing the cell adhesion area per unit region of the culture surface.

This enables ultrasonic vibration to act on the cell from all directions at the time of detachment using the application of ultrasonic vibration, thereby being able to improve the detachability. In addition, even at the time of culture, when the adhesion area is increased, it becomes difficult for the adhesion force to decrease, and hence high proliferation is obtained. Thus, it is possible to achieve both the detachability and the proliferation ability.

Further, the pitch P can fall within the range that allows a cell to easily enter the gaps 120 and to be sufficiently small relative to the size of a cell to be used, and the pitch P can satisfy 100 nm≤P≤1,000 nm.

In this embodiment, the recess/protrusion pattern height difference HD can satisfy 20 nm≤HD≤400 nm. When the recess/protrusion pattern height difference HD falls within this range, the surface area of the culture surface 10 is increased, and a cell easily reaches the lowest point of the recess/protrusion pattern and adheres thereto, thereby increasing the cell adhesion area per unit region of the culture surface. This enables vibration in an ultrasonic band to act on the cell from various directions at the time of detachment using the application of the vibration in an ultrasonic band, thereby being able to improve the detachability. Further, it becomes difficult for the adhesion force to decrease at the time of culture, and hence high proliferation is obtained. Thus, it is possible to achieve both the detachability and the proliferation ability.

In this embodiment, the recess/protrusion pattern height difference HD can satisfy 20 nm≤HD≤400 nm, and can satisfy 50 nm≤HD≤300 nm.

(Steps)

The cell detachment method and the cell manufacturing method according to this embodiment are illustrated in Fig. 4, both include the detachment step S320, and may further include the culture step S310 of culturing the cells, and the collection step S330 of collecting the detached cells. However, the culture step S310 and the collection step S330 are not essential. In regard to the respective steps, the description in the first embodiment can be referred to.

In the detachment step in this embodiment, the vibrating unit is caused to generate ultrasonic vibration under the state in which, when an observation image of a cross section perpendicular to the culture surface is obtained, the length Lcp of the contour of the cell on the culture surface side in the measurement region of the observation image and the length Lf of the measurement region in the direction parallel to the culture surface satisfy Lcp>Lf, and the ultrasonic vibration is transmitted to the cell, to thereby detach the cell from the culture surface.

It can cause, in the detachment step, the vibrating unit to generate ultrasonic vibration under a state in which Lcp/Lf≥1.2 is satisfied.

Further, it can cause, in the detachment step, the vibrating unit to generate ultrasonic vibration under a state in which a length Lp of a contour of the culture surface in the measurement region satisfies Lcp/Lp≥0.8.

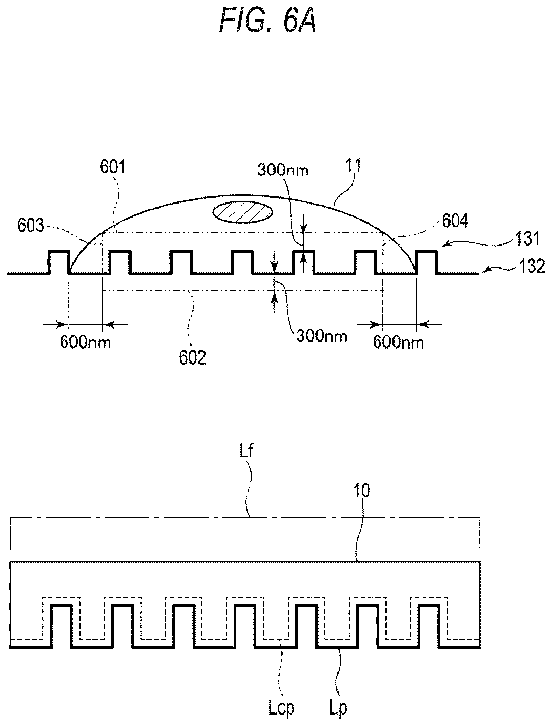

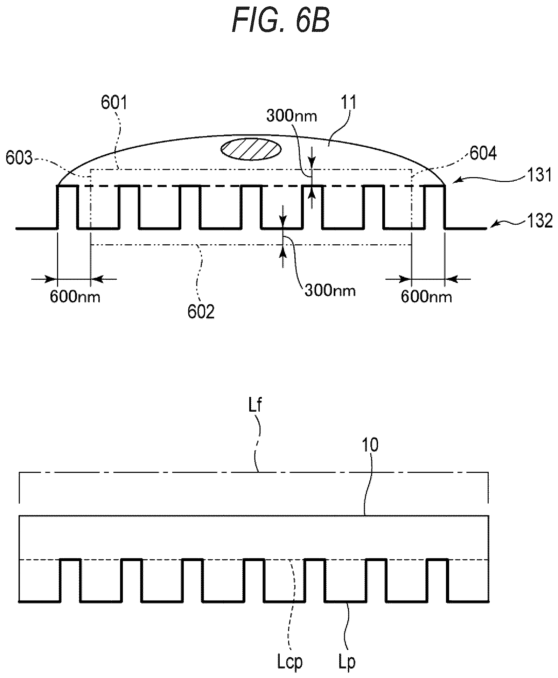

In each of Fig. 6A and Fig. 6B, a schematic view of a cell cultured in a culture vessel having a recessed/protruding surface is illustrated in the upper half, and an enlarged view of the culture surface is illustrated in the lower half. As illustrated in Fig. 6A and Fig. 6B, Lcp, Lf, and Lp are defined as the contour line Lcp (indicated by the dotted line in the figures) of the cell, the line Lf (indicated by the one-dot chain line in the figures) parallel to the culture surface, and the contour Lp (indicated by the solid line in the figures) of the culture surface, respectively. However, Lcp, Lf, and Lp are not required to be measured every time in the detachment step, and are verified, when required, by a verification method for Lcp, Lf, and Lp described later.

Lcp>Lf (state in which the contour of the cell on the culture surface side is longer than the length in the direction parallel to the culture surface) can refer to a state in which Lcp is at least 5% longer than Lf, that is, Lcp>Lf×1.05. Lcp being longer than Lf indicates that the cell adhesion area per unit region of the culture surface is larger than when the culture is performed on a related-art culture surface having no recess/protrusion pattern, and it is possible to achieve both the detachability and the proliferation ability of cells by performing the culture and the detachment using the application of vibration in an ultrasonic band in the above-mentioned state. Lcp and Lf can satisfy Lcp/Lf≥1.2, even more preferably satisfy Lcp/Lf≥1.7. Further, a cell can adhere to 30% or more of the height H or 30% or more of the depth D of the recessed/protruding portions forming the recess/protrusion pattern, and when the height H or the depth D falls within a range of from 400 nm to 1,000 nm inclusive, can simultaneously satisfy the formula Lcp/Lf>(height H [nm]/1,000+0.8) or the formula Lcp/Lf>(depth D [nm]/1,000+0.8).

Lcp and Lp can satisfy Lcp/Lp≥0.8. Lcp/Lp being large indicates that the cell has successfully followed the recess/protrusion pattern 100, and it is easy to achieve both the detachability and the proliferation ability by performing the culture and the detachment based on the ultrasonic vibration in the above-mentioned state. Lcp and Lp can satisfy Lcp/Lp≥0.9. As the cell more successfully follows the recess/protrusion pattern without a gap, the adhesion of the cell becomes more stable, and the proliferation is more easily maintained. In Fig. 6A, the cell has successfully followed the recess/protrusion pattern, and in Fig. 6B, the following has not been as successful as in Fig. 6A.

(Verification Method for Relationships of Lcp, Lf, and Lp)

In regard to the length Lcp of the contour of the cell on the culture surface side in the measurement region of the observation image, the length Lf of the measurement region in the direction parallel to the culture surface, and the length Lp of the contour of the culture surface in the measurement region, as described below, an observation image is acquired, Lcp, Lf, and Lp are determined based on the acquired observation image, and the Lcp, Lf, and Lp that have been determined are used to verify whether or not the respective relationships regarding those parameters are satisfied.

1) Acquisition of Observation Image

The observation image is an observation image of a cross section perpendicular to the culture surface (cross-sectional observation image). The cross-sectional observation image is obtained by observing a single cell and the cross section of a portion of the culture surface to which the cell adheres through use of a cryo-SEM, and a cross-sectional observation image in which an interface between the cell and the culture surface to which the cell adheres is clearly visible is to be used.

In order to obtain the cross-sectional observation image of the cell and the culture surface, it is required to immobilize the cell. A specific example of an operation method from immobilization of the cell to acquisition of the observation image is described below.

1-1) Immobilization

The medium is removed from the culture vessel to which the cells after culture adhere, and the cells are washed three times with PBS. 4% paraformaldehyde (FUJIFILM Wako Pure Chemical Corporation) is added to immobilize the cells. In the following Example, the cells were immobilized by being immersed in 4% paraformaldehyde for 15 minutes, but it is required to select an optimal immobilization time depending on the cell type. For example, when a clear observation image cannot be obtained due to cell deformation at the time of acquiring the observation image, the immobilization is insufficient or excessive, and hence the immersion time is adjusted.

1-2) Dehydration

The cells are dehydrated through use of a mixture of ethanol and pure water cooled to 4°C. First, the 4% paraformaldehyde used for immobilization is removed, and the cells are washed three times with PBS. A 50% aqueous solution of ethanol is added, and the resultant is left to stand for 10 minutes while being cooled at 4°C. The 50% aqueous solution of ethanol is removed, and a 60% aqueous solution of ethanol is then added, and the resultant is left to stand for 10 minutes while being cooled at 4°C. The same operation is performed through use of each of 70%, 80%, 90%, and 100% aqueous solutions of ethanol, and after the 100% aqueous solution of ethanol is removed, the resultant is left to stand overnight to dry.

1-3) Acquisition of Observation Image

The observation image is acquired by a cryo-SEM method using an FIB-SEM (Helios G4UC) manufactured by FEI. A cryo-temperature is set to -140°C, and a cross section is processed by an FIB, and then the cross section is observed. At this time, the cells for which observation images are to be acquired are not those immediately after division or densely clustered, but are in a firmly adhering state, and eight cells each appearing as only one cell within an angle of view or each having a clearly distinguishable boundary of one cell are randomly selected. A cross section is cut out by the FIB so as to pass near a center of each of the selected cells and near a center of the structure forming the recess/protrusion pattern, and a cross-sectional observation image is acquired by the SEM. At this time, the observation image is acquired in a direction in which the cell is positioned above the culture surface.

2) Determination of Lcp, Lf, and Lp

As illustrated in Fig. 6A and Fig. 6B, the contour line Lcp (indicated by the dotted line in the figures) of the cells, the line Lf (indicated by the one-dot chain line in the figures) parallel to the culture surface, and the contour Lp (indicated by the solid line in the figures) of the culture surface are measured to determine Lcp, Lf, and Lp, respectively.

Lcp, Lf, and Lp are measured by extracting a vicinity of an adhesion interface between the cell 11 and the culture surface 10 from the observation image as a measurement region. The measurement region is defined as an inner side of a rectangle in the observation image, the rectangle being formed by respective lines described below. In Fig. 6A and Fig. 6B, an upper side 601, a lower side 602, a left-hand side 603, and a right-hand side 604 are indicated by the two-dot chain lines.

Upper side 601: A line parallel to the culture surface 10 that passes 300 nm above the highest surface 131 at the adhesion interface between the cell 11 and the culture surface 10 in the observation image
Lower side 602: A line parallel to the culture surface that passes 300 nm below the lowest surface 132 of the recess/protrusion pattern in the observation image
Left-hand side 603: A line perpendicular to the culture surface that passes 600 nm to the right of the leftmost position of the adhesion interface between the cell 11 and the culture surface 10 in the observation image
Right-hand side 604: A line perpendicular to the culture surface that passes 600 nm to the left of the rightmost part of the adhesion interface between the cell 11 and the culture surface 10 in the observation image
Lcp is the length of a line on the culture surface side (bottom side) of the contour line of the cell 11 in a measurement region image. Lf is the length of the upper side or lower side of the measurement region image. Lp is the length of the line on the cell side (top side) of the contour line of the culture surface in the measurement region image.

Herein, being "parallel to the culture surface" means being parallel with respect to the culture surface. Being "parallel with respect to the culture surface" can be said to mean being parallel with respect to a surface assumed when the culture surface comprising the recess/protrusion pattern is viewed as a macroscopically smooth surface or being parallel with respect to the reference plane of the recess/protrusion pattern.

For each of the acquired observation images, Lcp, Lf, and Lp are measured, and average values thereof are calculated to be set as Lcp, Lf, and Lp.

3) Verification

The relationship between Lcp and Lf, or Lcp/Lp, is obtained based on the Lcp, Lf, and Lp that have been determined.

(Examples)

The present disclosure is described below in more detail below by way of Examples and Comparative Examples.

However, the present disclosure is by no means limited thereto.

The cells to be used in Examples and the like of the present disclosure were each obtained in accordance with the culture conditions shown below.

(Culture of CHO Cells)

Chinese hamster ovary (CHO) cells were seeded in a $\Phi$35 polystyrene dish (manufactured by Corning Incorporated) at a density of 10,000 cells/cm$^2$, and cultured under an environment at 37°C and a $CO_2$ concentration of 5%. Ham's F12 (manufactured by Thermo Fisher Scientific) supplemented with 10% of Fetal Bovine Serum (manufactured by Sigma-Aldrich) and 1% of penicillin-streptomycin (10,000 U/ml, manufactured by Thermo Fisher Scientific) was used as a medium. The culture was performed for 48 hours, and the state of the cells was observed with a phase contrast microscope to recognize cell adhesion and proliferation. The cell coverage area ratio of the dish was about 80%.

(Culture of MDCK Cells)

MDCK cells (canine renal tubular epithelial cells) were seeded in a $\Phi$35 polystyrene dish (manufactured by Corning Incorporated) at a density of 10,000 cells/cm$^2$, and cultured under an environment at 37°C and a $CO_2$ concentration of 5%. Eagles's Minimum Essential Medium (FUJIFILM Wako Pure Chemical) supplemented with 10% of Fetal Bovine Serum (manufactured by Sigma-Aldrich) and 1% of penicillin-streptomycin (10,000 U/ml, manufactured by Thermo Fisher Scientific) was used as a medium. The culture was performed for 48 hours, and the state of the cells was observed with a phase contrast microscope to recognize cell adhesion and proliferation. The cell coverage area ratio of the dish was about 80%.

(Culture of A549 Cells)

A549 cells (human alveolar basal epithelial adenocarcinoma cells) were seeded in a $\Phi$35 polystyrene dish (manufactured by Corning Incorporated) at a density of 10,000 cells/cm$^2$, and cultured under an environment at 37°C and a $CO_2$ concentration of 5%. DMEM (manufactured by Thermo Fisher Scientific) supplemented with 10% of Fetal Bovine Serum (manufactured by Sigma-Aldrich) and 1% of penicillin-streptomycin (10,000 U/ml, manufactured by Thermo Fisher Scientific) was used as a medium. The culture was performed for 48 hours, and the state of the cells was observed with a phase contrast microscope to recognize cell adhesion and proliferation. The cell coverage area ratio of the dish was about 80%.

(Culture of MSC Cells)

MSC cells (mesenchymal stem cells) were seeded in a $\Phi$35 polystyrene dish (manufactured by Corning Incorporated) at a density of 4,000 cells/cm$^2$, and cultured under an environment at 37°C and a $CO_2$ concentration of 5%. Mesenchymal Stem Cell Growth Medium 2 (manufactured by PromoCell) supplemented with 10% of Fetal Bovine Serum (manufactured by Sigma-Aldrich) and 1% of penicillin-streptomycin (10,000 U/ml, manufactured by Thermo Fisher Scientific) was used as a medium. The culture was performed for 168 hours with the medium exchanged 72 hours after the start of the culture, and the state of the cells was observed with a phase contrast microscope to recognize cell adhesion and proliferation. The cell coverage area ratio of the dish was about 80%.

(Culture of Mouse Macrophage-like Cells)

Mouse macrophage-like cells (RAW264.7) were seeded in a $\Phi$35 polystyrene dish (manufactured by Corning Incorporated) at a density of 20,000 cells/cm$^2$, and cultured under an environment at 37°C and a $CO_2$ concentration of 5%. DMEM (manufactured by Thermo Fisher Scientific) supplemented with 10% of Fetal Bovine Serum (manufactured by Sigma-Aldrich) and 1% of penicillin-streptomycin (10,000 U/ml, manufactured by Thermo Fisher Scientific) was used as a medium. The culture was performed for 48 hours, and the state of the cells was observed with a phase contrast microscope to recognize cell adhesion and growth. The cell coverage area ratio of the dish was about 80%.

(Detachment Unit using Application of Vibration in Ultrasonic Band)

The culture vessel was set on the ultrasonic device through use of the vibration application mechanism illustrated in Fig. 5 which has been described above. After that, at an environmental temperature of 37°C, respective ultrasonic devices and vibration application conditions shown in Table 1 were used to perform driving to apply vibration in an ultrasonic band.

When a ring device is used as an ultrasonic device in a standing wave mode, it is possible to apply such bending vibration in a vibration mode that the maximum amplitude acts on a central portion of the culture surface of the culture vessel in the frequency band that has been used.

When a Langevin device is used as the ultrasonic device, vibration having a nearly flat vibration distribution with the maximum amplitude in the central portion can be applied to the entire culture surface of the culture vessel.

Table 1

| | Ultrasonic device | Vibration application conditions | | |
|---|---|---|---|---|
| | | Output voltage (V) | Frequency (kHz) | Sweep period (s) |
| Condition 1 | Ring device (standing wave mode) | 30 | 29-35 | 1 |
| Condition 2 | Ring device (standing wave mode) | 50 | 29-35 | 1 |
| Condition 3 | Ring device (standing wave mode) | 100 | 29-35 | 1 |
| Condition 4 | Langevin device | 20 | 35-37 | 1 |

(Jet Detachment Unit)

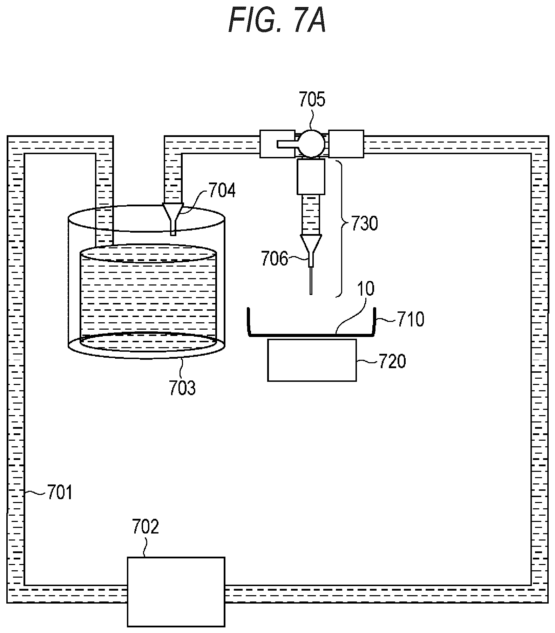

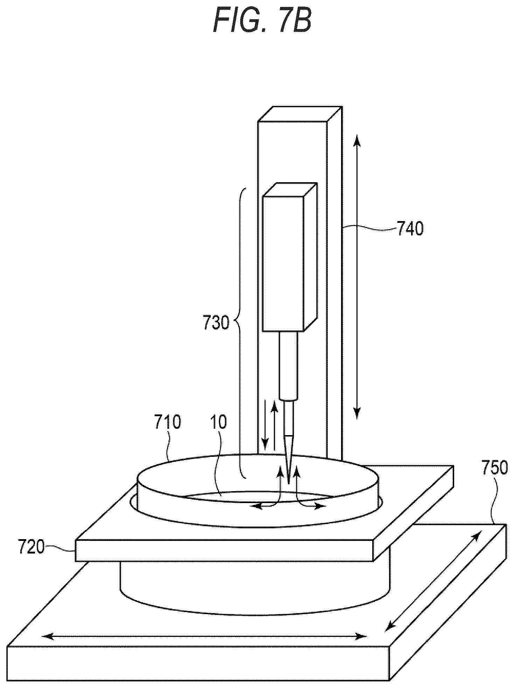

A jet detachment apparatus illustrated in Fig. 7A and Fig. 7B was used to perform detachment based on a jet without use of the ultrasonic vibration.

As illustrated in Fig. 7A, the jet detachment apparatus was produced by combining a silicone tube 701, a tube pump 702, a reservoir tank 703, a needle 704, a three-way valve 705, and a jet mechanism 730 comprising a nozzle 706. A culture vessel 710, a holding mechanism 720 that holds the culture vessel 710, a support column 740, and a base 750 are illustrated in Fig. 7A and Fig. 7B. Then, the culture vessel 710 is placed as illustrated in Fig. 7B. The culture vessel 710 in which cells (not shown in Fig. 7A and Fig. 7B) had been cultured was arranged such that a distance between a distal end of the nozzle 706 having an inner diameter of 1.04 mm and the culture surface 10 was 17 mm, and PBS was sprayed over the entire culture surface from the nozzle 706 at 0.65 mL/s perpendicularly to the culture surface for 3 seconds, to thereby perform the detachment based on a jet.

(Preparation of Culture Vessel 1)

A Φ (diameter) 35mm polystyrene dish (manufactured by Corning Incorporated) was used as a base of the culture vessel. A mold (nickel) corresponding to the recess/protrusion pattern 100 was pressed against the culture vessel softened by being heated to 95°C, to thereby transfer the recess/protrusion pattern onto the culture surface. The pressing was performed under the condition that the pressure was increased to 40 MPa at 10 MPa/min and then maintained at 40 MPa for 3 minutes. Immediately after the pressing, the culture vessel was cooled and released from the mold, to thereby obtain a culture vessel 1.

The obtained culture vessel 1 was washed and sterilized with ethanol, and then used for culture.

(Preparation of Culture Vessels 2 to 30)

Culture vessels 2 to 30 were produced in the same manner as in the case of the culture vessel 1 except that the recess/protrusion pattern was changed to corresponding ones shown in Table 2. A list of the culture vessels is shown in Table 2.

(Culture vessel 31)

A Φ35mm polystyrene dish (manufactured by Corning Incorporated) was used for a related-art culture vessel having no recess/protrusion pattern, to thereby obtain a culture vessel 31.

Table 2

| Culture vessel | Details of recess/protrusion pattern | | | | | |
|---|---|---|---|---|---|---|
| | Recessed/protruding portion | Pitch P (nm) | Height H or depth D (nm) | Interval I or hole diameter S (nm) | Presence or absence of curved surface portion | H/I or D/S |
| 1 | Cylindrical protruding portion | 400 | 200 | 200 | Present | 1.00 |
| 2 | Cylindrical protruding portion | 400 | 20 | 200 | Absent | 0.10 |
| 3 | Cylindrical protruding portion | 400 | 100 | 100 | Present | 1.00 |
| 4 | Cylindrical protruding portion | 400 | 300 | 300 | Present | 1.00 |
| 5 | Cylindrical protruding portion | 600 | 400 | 400 | Present | 1.00 |
| 6 | Cylindrical protruding portion | 600 | 500 | 400 | Present | 1.25 |
| 7 | Cylindrical protruding portion | 400 | 50 | 200 | Present | 0.25 |
| 8 | Cylindrical protruding portion | 100 | 50 | 50 | Present | 1.00 |
| 9 | Cylindrical protruding portion | 1,000 | 200 | 800 | Present | 0.25 |
| 10 | Cylindrical protruding portion | 2,000 | 200 | 1,800 | Present | 0.11 |
| 11 | Cylindrical protruding portion | 400 | 330 | 300 | Present | 1.10 |
| 12 | Cylindrical protruding portion | 400 | 200 | 200 | Absent | 1.00 |
| 13 | Cylindrical recessed portion | 400 | 200 | 200 | Present | 1.00 |
| 14 | Cylindrical protruding portion | 400 | 400 | 300 | Present | 1.20 |
| 15 | Cylindrical protruding portion | 500 | 300 | 300 | Present | 1.00 |
| 16 | Cylindrical protruding portion | 300 | 100 | 100 | Present | 1.00 |
| 17 | Cylindrical protruding portion | 600 | 200 | 400 | Present | 0.50 |
| 18 | Cylindrical protruding portion | 800 | 400 | 500 | Present | 0.80 |
| 19 | Cylindrical protruding portion | 800 | 600 | 500 | Present | 1.20 |
| 20 | Cylindrical protruding portion | 800 | 800 | 500 | Present | 1.60 |
| 21 | Cylindrical protruding portion | 800 | 1,000 | 500 | Present | 2.00 |
| 22 | Cylindrical protruding portion | 800 | 1,000 | 300 | Present | 3.33 |
| 23 | Cylindrical protruding portion | 1,000 | 65 | 700 | Present | 0.09 |
| 24 | Cylindrical protruding portion | 900 | 800 | 500 | Present | 1.14 |
| 25 | Cylindrical protruding portion | 900 | 800 | 300 | Present | 2.67 |
| 26 | Cylindrical protruding portion | 50 | 600 | 20 | Present | 30.00 |
| 27 | Cylindrical protruding portion | 800 | 1,500 | 600 | Present | 2.50 |
| 28 | Cylindrical protruding portion | 3,000 | 600 | 2,800 | Present | 0.18 |
| 29 | Cylindrical protruding portion | 600 | 3,000 | 300 | Present | 10.00 |
| 30 | Cylindrical protruding portion | 300 | 450 | 150 | Present | 3.00 |
| 31 | No recess/protrusion pattern ("related art culture vessel") | | | | | |

(Evaluation Criteria for Detachability)

The related-art culture vessel (culture vessel 31) having no recess/protrusion pattern and each culture vessel according to the present disclosure having a recess/protrusion pattern were compared by culturing cells therein under the same culture conditions and also detaching the cultured cells under the same detachment conditions. A proportion of the detached cells was set as a detachment ratio, and the detachment ratios of the respective vessels were compared to evaluate the detachability. The evaluation criteria were as follows.

A: 180% or more of the detachment ratio obtained through use of the related-art culture vessel
B: 150% or more and less than 180% of the detachment ratio obtained through use of the related-art culture vessel
C: 120% or more and less than 150% of the detachment ratio obtained through use of the related-art culture vessel
D: Less than 120% of the detachment ratio obtained through use of the related-art culture vessel

(Evaluation Criteria for Proliferation ability)

The related-art culture vessel (culture vessel 31) having no recess/protrusion pattern and each culture vessel according to the present disclosure having a recess/protrusion pattern were used to culture cells therein under the same culture conditions. A ratio between the number of seeded cells and the number of cells after the culture was set as the proliferation ratio, and the proliferation ratios of the respective culture vessels were compared to evaluate the proliferation ability. The evaluation criteria were as follows.

A: 90% or more of the proliferation ratio obtained through use of the related-art culture vessel

B: 80% or more and less than 90% of the proliferation ratio obtained through use of the related-art culture vessel

C: 70% or more and less than 80% of the proliferation ratio obtained through use of the related-art culture vessel

D: Less than 70% of the proliferation ratio obtained through use of the related-art culture vessel

(Evaluation Criteria for Initial Adhesive Property)

The related-art culture vessel (culture vessel 31) having no recess/protrusion pattern and each culture vessel according to the present disclosure having a recess/protrusion pattern were used to culture cells therein under the same culture conditions and also detach the cells under the same detachment conditions. The cells collected by the detachment were re-seeded in the related-art culture vessel having no recess/protrusion pattern, and left to stand for 1 hour. A proportion of cells that adhered after standing was set as an initial adhesion ratio, and the initial adhesion ratios of the respective vessels were compared to evaluate the initial adhesion ability. The evaluation criteria were as follows.

A: The initial adhesion ratio is 200% or more of the initial adhesion ratio obtained through use of the related-art culture vessel.
B: The initial adhesion ratio is 150% or more and less than 200% of the initial adhesion ratio obtained through use of the related-art culture vessel.
C: The initial adhesion ratio is 95% or more and less than 150% of the initial adhesion ratio obtained through use of the related-art culture vessel.
D: The initial adhesion ratio is less than 95% of the initial adhesion ratio obtained through use of the related-art culture vessel.

(Evaluation Criteria for Differentiation Potency)

The related-art culture vessel (culture vessel 31) having no recess/protrusion pattern and each culture vessel according to the present disclosure having a recess/protrusion pattern were used to culture mesenchymal stem cells therein under the same culture conditions and also detach the mesenchymal stem cells under the same detachment conditions. The cells collected by the detachment were used to perform differentiation into cartilage. Diameters of respective cell aggregates after the differentiation were compared to evaluate cartilage differentiation ability. The evaluation criteria were as follows.

A: The diameter of the obtained cell aggregates is 130% or more of the diameter of the cell aggregates obtained through use of the related-art culture vessel.

B: The diameter of the obtained cell aggregates is 120% or more and 130% or less of the diameter of the cell aggregates obtained through use of the related-art culture vessel.

C: The diameter of the obtained cell aggregates is 105% or more and 120% or less of the diameter of the cell aggregates obtained through use of the related-art culture vessel.

D: The diameter of the obtained cell aggregates is 105% or less of the diameter of the cell aggregates obtained through use of the related-art culture vessel.

(Example 1 and Comparative Example 1)

In Example 1, CHO cells were cultured in the culture vessel 1, and in Comparative Example 1, CHO cells were cultured in the culture vessel 31. For the culture, four culture vessels 1 were used in Example 1, and three culture vessels 31 were used in Comparative Example 1, to thereby respectively culture the CHO cells under the same conditions. One of the culture vessels 1 was used for cross-sectional observation, and the cells in the remaining vessels were used to evaluate the detachability, the proliferation ability, and the initial adhesion ability.

(Cross-sectional Observation)

In Example 1, the observation images of cells and recess/protrusion patterns were acquired by the above-mentioned "Verification Method for Relationship of Lcp, Lf, and Lp" to determine Lcp, Lf, and Lp and obtain Lcp/Lf and Lcp/Lp. Lcp/Lf was 1.8, and Lcp/Lp was 1.0.

(Evaluation of Detachability and Proliferation ability)

After the cell culture was completed, the medium in each culture vessel was removed, and the cells were washed with PBS(-), and then immersed in PBS(-) serving as a detachment solution for 3 minutes. After that, an ultrasonic transducer was brought into contact with the culture vessel to apply vibration in an ultrasonic band at an environmental temperature of 37°C. The ultrasonic vibration was applied for 5 minutes through use of a ring device as a transducer with sweep vibration in the standing wave mode (frequency of from 29 kHz to 35 kHz, sweep period of 1 second, and voltage of 30 V). After the detached cells were collected, the number of cells was measured through use of a hemocytometer, and the cell viability was calculated through use of a viability determination method based on trypan blue staining. Further, all cells that was not able to be detached by the ultrasonic vibration were also detached through use of a cell scraper from the culture vessel after the detachment based on the ultrasonic vibration, and the number of cells was measured through use of the hemocytometer. The number of cells detached by the ultrasonic vibration and the number of cells detached by the cell scraper thereafter were added up to obtain a total number of cells, the detachment ratio was calculated by Equation 1, and the proliferation ratio was calculated by Equation 2. The three culture vessels 1 and the three culture vessels 31 were used to calculate the detachment ratios and proliferation ratios, and an average value for each vessel type was used.

$$\text{detachment ratio (\%)}=\text{(number of cells detached by ultrasonic vibration)}/\text{(total number of cells)}\times 100 \qquad \text{Equation 1:}$$

$$\text{proliferation ratio (\%)}=\text{(total number of cells)}/\text{(number of seeded cells)}\times 100 \qquad \text{Equation 2:}$$

An average detachment ratio of the cells detached from the culture vessel 1 by the ultrasonic vibration was 80.0%, and was 212.8% of the average detachment ratio of the cells detached from the culture vessel 31. An average proliferation ratio of the cells cultured in the culture vessel 1 was 663%, and was 107.5% of the average proliferation ratio of the cells cultured in the culture vessel 31. In addition, the viability of the cells detached by the ultrasonic vibration was 99% or more in all cases.

(Evaluation of Initial Adhesion Ability)

The cells detached from the culture vessel 1 and the culture vessel 31 by the ultrasonic vibration were seeded in a new culture vessel 31 to a density of 35,000 cells/cm$^2$. The cells were homogenized by being gently shaken and left to stand for 1 hour in a CO$_2$ incubator at 37°C. After standing, the medium was slowly sucked out from the culture vessel, and the cells that had not adhered were collected, and counted through use of the hemocytometer. Further, the cells remaining in the culture vessel were collected by the cell scraper, and counted through use of the hemocytometer. The initial adhesion ratio was calculated by Equation 3. The initial adhesion ratios were calculated through use of the cells collected from each of the three culture vessels 1 and the three culture vessels 31, and an average value for each vessel type was used.

$$\text{initial adhesion ratio (\%)}=\text{(number of cells that have adhered within 1 hour)}/\text{((number of cells that have not adhered)}+\text{(number of cells that have adhered within 1 hour))} \qquad \text{Equation 3:}$$

An average initial adhesion ratio of the cells collected from the culture vessel 1 was 65%, and was 244% of the average initial adhesion ratio of the cells collected from the culture vessel 31.

(Examples 2 to 7)

The detachability, the proliferation ability, and the initial adhesion ability were evaluated in the same manner as in Example 1 except that the culture vessel 1 was changed to corresponding ones shown in Table 3-1 and Table 3-2. Results thereof are shown in Table 3-1 and Table 3-2.

(Examples 8 to 33)

The detachability and the proliferation ability were evaluated in the same manner as in Example 1 except that the cell type, the culture vessel, and the ultrasonic vibration application conditions were changed to corresponding ones shown in Table 3-1 and Table 3-2. Results thereof are shown in Table 3-1 and Table 3-2.

(Comparative Example 2)

The detachability and the proliferation ability were evaluated in the same manner as in Example 1 except that the cell type and the culture vessel were changed to corresponding ones shown in Table 3-1 and Table 3-2 and the jet detachment apparatus illustrated in Fig. 7A and Fig. 7B was used instead of the application of the ultrasonic vibration. Results thereof are shown in Table 3-1 and Table 3-2.

(Comparative Examples 3 to 7)

The detachability and the proliferation ability were evaluated in the same manner as in Example 1 except that the cell type, the culture vessel, and the ultrasonic vibration application conditions were changed to corresponding ones shown in Table 3-1 and Table 3-2. Results thereof are shown in Table 3-1 and Table 3-2.

Table 3-1

|  | Cell type | Culture vessel | Ultrasonic conditions | Lcp/Lf | Lcp/Lp |
|---|---|---|---|---|---|
| Example 1 | CHO | 1 | 1 | 1.8 | 1.0 |
| Example 2 | CHO | 2 | 1 | 1.1 | 1.0 |
| Example 3 | CHO | 3 | 1 | 1.4 | 1.0 |
| Example 4 | CHO | 4 | 1 | 2.1 | 0.9 |
| Example 5 | CHO | 5 | 1 | 1.6 | 0.8 |
| Example 6 | CHO | 6 | 1 | 1.5 | 0.6 |
| Example 7 | CHO | 7 | 1 | 1.2 | 1.0 |
| Example 8 | CHO | 8 | 1 | 1.3 | 0.7 |
| Example 9 | CHO | 9 | 1 | 1.3 | 1.0 |
| Example 10 | CHO | 10 | 1 | 1.1 | 0.9 |
| Example 11 | CHO | 11 | 1 | 2.0 | 0.8 |
| Example 12 | CHO | 12 | 1 | 1.8 | 0.9 |
| Example 13 | CHO | 13 | 1 | 1.8 | 1.0 |
| Example 14 | CHO | 14 | 1 | 1.6 | 0.6 |
| Example 15 | MSC | 1 | 2 | 1.8 | 1.0 |
| Example 16 | MSC | 15 | 2 | 1.9 | 1.0 |
| Example 17 | MSC | 16 | 2 | 1.6 | 1.0 |
| Example 18 | MSC | 12 | 2 | 1.9 | 0.9 |
| Example 19 | C2C12 | 17 | 3 | 1.6 | 1.0 |

(continued)

|  | Cell type | Culture vessel | Ultrasonic conditions | Lcp/Lf | Lcp/Lp |
|---|---|---|---|---|---|
| Example 20 | C2C12 | 18 | 3 | 1.8 | 1.0 |
| Example 21 | C2C12 | 19 | 3 | 2.1 | 0.9 |
| Example 22 | C2C12 | 20 | 3 | 2.1 | 0.8 |
| Example 23 | C2C12 | 21 | 3 | 2.0 | 0.6 |
| Example 24 | C2C12 | 22 | 3 | 1.7 | 0.5 |
| Example 25 | C2C12 | 23 | 3 | 1.1 | 1.0 |
| Example 26 | C2C12 | 24 | 3 | 1.8 | 0.7 |
| Example 27 | C2C12 | 25 | 3 | 1.5 | 0.5 |
| Example 28 | MDCK | 19 | 3 | 2.2 | 1.0 |
| Example 29 | MDCK | 1 | 3 | 1.8 | 1.0 |
| Example 30 | A549 | 19 | 2 | 2.1 | 0.9 |
| Example 31 | A549 | 1 | 2 | 1.8 | 1.0 |
| Example 32 | MΦ | 1 | 3 | 1.8 | 1.0 |
| Example 33 | CHO | 1 | 4 | 1.8 | 1.0 |
| Comparative Example 1 | CHO | 31 | 1 | 1.0 | 1.0 |
| Comparative Example 2 | C2C12 | 19 | Jet | 2.1 | 0.9 |
| Comparative Example 3 | C2C12 | 26 | 3 | 1.0 | 0.0 |
| Comparative Example 4 | C2C12 | 27 | 3 | 1.0 | 0.2 |
| Comparative Example 5 | CHO | 28 | 1 | 1.0 | 0.8 |
| Comparative Example 6 | C2C12 | 29 | 3 | 1.0 | 0.1 |
| Comparative Example 7 | CHO | 30 | 1 | 1.0 | 0.3 |

Table 3-2

|  | Detachability (%) | | Proliferation potential (%) | | Initial adhesive property (%) | |
|---|---|---|---|---|---|---|
| Example 1 | 213 | A | 108 | A | 244 | A |
| Example 2 | 120 | C | 102 | A | 147 | C |
| Example 3 | 175 | B | 105 | A | 235 | A |
| Example 4 | 217 | A | 95 | B | 205 | A |
| Example 5 | 200 | A | 84 | C | 186 | B |
| Example 6 | 202 | A | 74 | C | 122 | C |
| Example 7 | 168 | B | 104 | A | 221 | A |
| Example 8 | 175 | B | 76 | C | - | |
| Example 9 | 175 | B | 104 | A | - | |
| Example 10 | 130 | C | 98 | A | - | |
| Example 11 | 202 | A | 88 | C | - | |
| Example 12 | 205 | A | 95 | B | - | |
| Example 13 | 207 | A | 107 | A | - | |
| Example 14 | 196 | A | 70 | C | - | |
| Example 15 | 205 | A | 107 | A | - | |

(continued)

|  | Detachability (%) |  | Proliferation potential (%) |  | Initial adhesive property (%) |  |
|---|---|---|---|---|---|---|
| Example 16 | 209 | A | 102 | A | - |  |
| Example 17 | 178 | B | 105 | A | - |  |
| Example 18 | 206 | A | 99 | C | - |  |
| Example 19 | 180 | A | 105 | A | - |  |
| Example 20 | 200 | A | 107 | A | - |  |
| Example 21 | 222 | A | 98 | A | - |  |
| Example 22 | 226 | A | 82 | B | - |  |
| Example 23 | 230 | A | 72 | C | - |  |
| Example 24 | 205 | A | 70 | C | - |  |
| Example 25 | 125 | C | 101 | A | - |  |
| Example 26 | 195 | A | 77 | C | - |  |
| Example 27 | 197 | A | 71 | C | - |  |
| Example 28 | 203 | A | 102 | B | - |  |
| Example 29 | 200 | A | 107 | A | - |  |
| Example 30 | 199 | A | 98 | C | - |  |
| Example 31 | 195 | A | 104 | A | - |  |
| Example 32 | 193 | A | 104 | A | - |  |
| Example 33 | 220 | A | 108 | A | - |  |
| Comparative Example 1 | 100 | D | 100 | A | - |  |
| Comparative Example 2 | 85 | D | 98 | A | - |  |
| Comparative Example 3 | 180 | A | 54 | D | - |  |
| Comparative Example 4 | 181 | A | 60 | D | - |  |
| Comparative Example 5 | 105 | D | 82 | A | - |  |
| Comparative Example 6 | 195 | A | 58 | D | - |  |
| Comparative Example 7 | 210 | A | 65 | D | - |  |

(Evaluation of Cartilage Differentiation Ability in Example 15)

In Example 15, the differentiation ability into cartilage was evaluated through use of the cells after the detachment based on the ultrasonic vibration. Differentiation into cartilage was performed by preparing pellets from 200,000 cells of each type of cell collected from each culture vessel and culturing the cells in a 15 mL polypropylene tube for 504 hours. A cartilage differentiation medium (manufactured by PromoCell) containing 1% TGF-$\beta$3 was used as a medium, and the medium was exchanged once every three days. The cell aggregates after the differentiation was taken out, and an observation image was acquired through use of the phase contrast microscope. The diameter of the cell aggregates was measured from the obtained observation image, and the differentiation ability was evaluated in accordance with the above-mentioned evaluation criteria. The diameters of the cell aggregates were measured through use of the cells collected from each of the three culture vessels 1 and the three culture vessels 31, and an average value for each vessel type was used.

The diameter of the cell aggregates collected from the culture vessel 1 and subjected to cartilage differentiation was 1,300 $\mu$m, and was 140% of the diameter of the cell aggregates collected from the culture vessel 31.

(Evaluation of Cartilage Differentiation Ability in Examples 16 to 18)

In Examples 16 to 18, the cartilage differentiation ability was evaluated in the same manner as in Example 15 except that the culture vessel was changed to ones shown in Table 4. Results thereof are shown in Table 4.

Table 4

| | Cell type | Culture vessel | Presence or absence of curved surface portion | Diameter of cell aggregates ($\mu$m) | Differentiation potency | |
|---|---|---|---|---|---|---|
| Example 15 | MSC | 1 | Present | 1,300 | 140 | A |
| Example 16 | MSC | 15 | Present | 1,198 | 129 | B |
| Example 17 | MSC | 16 | Present | 1,207 | 132 | A |
| Example 18 | MSC | 12 | Absent | 975 | 105 | C |

Thus, it has been indicated that, among the culture vessels according to the present disclosure, cells cultured in the culture vessel with the recess/protrusion pattern having curved surface portions and then detached by the application of the vibration in an ultrasonic band have higher differentiation potency into cartilage than the cells cultured through use of the culture vessel with the recess/protrusion pattern having no curved surface portions and detached therefrom. The inventors consider that this is because the recess/protrusion pattern having the curved surface portions may have created an environment of scaffold similar to that *in vivo,* thereby leading to increased production of extracellular matrix. Through use of the cells collected in the present disclosure, differentiation may become more efficient, and it may also be possible to efficiently supply extracellular matrix that is highly valuable as a research subject.

Various embodiments have been described in detail above but it will be understood that the present disclosure is not limited to these embodiments and encompasses all modifications, variants, alternatives and equivalents falling within the scope of the appended claims.

**Claims**

1.    A cell detachment method comprising:

providing a culture vessel (421) having a periodic recess/protrusion pattern on a culture surface, wherein one or more cells are adhered to the culture surface from the culture surface,
causing a vibrating unit arranged and configured to apply vibration to the culture vessel (421) to generate ultrasonic vibration under a state in which, when an observation image of a cross section perpendicular to the culture surface is obtained, a length Lcp of a contour of the at least one cell on the culture surface side in a measurement region of the observation image and a length Lf of the measurement region in a direction parallel to the culture surface satisfy

$$Lcp > Lf;$$

and
transmitting the ultrasonic vibration to the at least one cell, thereby detaching the at least one cell from the culture surface.

2.    The cell detachment method according to claim 1, wherein a pitch P of the periodic recess/protrusion pattern satisfies

$$100 \text{ nm} \leq P \leq 2{,}000 \text{ nm}.$$

3.    The cell detachment method according to claim 1 or 2, wherein a recess/protrusion pattern height difference HD of the periodic recess/protrusion pattern satisfies

$$20 \text{ nm} \leq HD \leq 400 \text{ nm}.$$

4.    The cell detachment method according to any one of claims 1 to 3, wherein the periodic recess/protrusion pattern comprises at least any one of the following (A) or (B):

(A) a plurality of protruding portions which protrude from a reference plane of the periodic recess/protrusion pattern, and are periodically arranged, where:
the plurality of protruding portions are arranged at a height H and an interval I; and

$$20\ \text{nm} \leq H \leq 400\ \text{nm and } H/I \leq 1.1$$

are satisfied; and

(B) a plurality of recessed portions which are recessed from the reference plane of the periodic recess/protrusion pattern, and are periodically arranged, where:

the plurality of recessed portions have a depth D and a hole diameter S; and

$$20\ \text{nm} \leq D \leq 400\ \text{nm and } D/S \leq 1.1$$

are satisfied.

5. The cell detachment method according to any one of claims 1 to 4, wherein the detachment step comprises causing the vibrating unit to generate ultrasonic vibration under a state in which

$$Lcp/Lf \geq 1.2$$

is satisfied.

6. The cell detachment method according to any one of claims 1 to 5, wherein the detachment step comprises causing the vibrating unit to generate ultrasonic vibration under a state in which a length Lp of a contour of the culture surface in the measurement region satisfies

$$Lcp/Lp \geq 0.8.$$

7. The cell detachment method according to any one of claims 1 to 6, wherein the detachment step comprises applying, by the vibrating unit, vibration from a back side of the culture surface.

8. A cell detachment method comprising:

providing a culture vessel (421) having a periodic recess/protrusion pattern on a culture surface, wherein one or more cells are adhered to the culture surface from the culture surface,
causing a vibrating unit arranged and configured to apply vibration to the culture vessel (421) to generate ultrasonic vibration; and
transmitting the ultrasonic vibration to the cell, to thereby detach the cell from the culture surface,
wherein a pitch P of the periodic recess/protrusion pattern on the culture surface satisfies

$$100\ \text{nm} \leq P \leq 2{,}000\ \text{nm},$$

and
wherein the culture surface comprises at least any one of the following (A) or (B):

(A) a plurality of protruding portions which protrude from a reference plane of the periodic recess/protrusion pattern, and are periodically arranged, where:
the plurality of protruding portions are arranged at a height H and an interval I; and

$$20\ \text{nm} \leq H \leq 400\ \text{nm and } H/I \leq 1.1$$

are satisfied; and

(B) a plurality of recessed portions which are recessed from the reference plane of the periodic recess/protrusion pattern, and are periodically arranged, where:
the plurality of recessed portions have a depth D and a hole diameter S; and

$$20\ \text{nm} \leq D \leq 400\ \text{nm and } D/S \leq 1.1$$

are satisfied.

9. The cell detachment method according to claim 8, wherein a recess/protrusion pattern height difference HD of the periodic recess/protrusion pattern satisfies

$$20 \text{ nm} \leq HD \leq 400 \text{ nm}.$$

10. The cell detachment method according to claim 8 or 9, wherein the detachment step comprises applying, by the vibrating unit, vibration from a back side of the culture surface.

11. A cell manufacturing method comprising:

culturing at least one cell in a culture vessel (421) having a periodic recess/protrusion pattern on a culture surface; and
detaching the at least one cell adhering to the culture surface from the culture surface,
wherein detaching the at least one cell comprises:

causing a vibrating unit arranged and configured to apply vibration to the culture vessel (421) to generate ultrasonic vibration under a state in which, when an observation image of a cross section perpendicular to the culture surface is obtained, a length Lcp of a contour of the cell on the culture surface side in a measurement region of the observation image and a length Lf of the measurement region in a direction parallel to the culture surface satisfy

$$Lcp > Lf;$$

and
transmitting the ultrasonic vibration to the cell, to thereby detach the cell from the culture surface.

12. A cell manufacturing method comprising:

culturing at least one cell in a culture vessel (421) having a periodic recess/protrusion pattern on a culture surface; and
detaching the at least one cell adhering to the culture surface from the culture surface,
wherein detaching the at least one cell comprises:

causing a vibrating unit arranged and configured to apply vibration to the culture vessel (421) to generate ultrasonic vibration; and
transmitting the ultrasonic vibration to the at least one cell, to thereby detach the at least one cell from the culture surface,

wherein a pitch P of the periodic recess/protrusion pattern on the culture surface satisfies

$$100 \text{ nm} \leq P \leq 2{,}000 \text{ nm},$$

and
wherein the periodic recess/protrusion pattern comprises at least any one of the following (A) or (B):

(A) a plurality of protruding portions (101) which protrude from a reference plane of the periodic recess/protrusion pattern, and are periodically arranged, where:
the plurality of protruding portions (101) are arranged at a height H and an interval I; and

$$20 \text{ nm} \leq H \leq 400 \text{ nm and } H/I \leq 1.1$$

are satisfied; and
(B) a plurality of recessed portions (102) which are recessed from the reference plane of the periodic recess/protrusion pattern, and are periodically arranged, where:
the plurality of recessed portions (102) have a depth D and a hole diameter S; and

$$20 \text{ nm} \leq D \leq 400 \text{ nm and } D/S \leq 1.1$$

are satisfied.

13. A cell culture vessel (421) comprising a periodic recess/protrusion pattern on a culture surface,

wherein a pitch P of the periodic recess/protrusion pattern on the culture surface satisfies

$$100 \text{ nm} \leq P \leq 2{,}000 \text{ nm,}$$

and
wherein the periodic recess/protrusion pattern comprises at least any one of the following (A) or (B):

(A) a plurality of protruding portions (101) which protrude from a reference plane of the periodic recess/protrusion pattern, and are periodically arranged, where:
the plurality of protruding portions (101) are arranged at a height H and an interval I; and

$$20 \text{ nm} \leq H \leq 400 \text{ nm and } H/I \leq 1.1$$

are satisfied; and
(B) a plurality of recessed portions (102) which are recessed from the reference plane of the periodic recess/protrusion pattern, and are periodically arranged, where:
the plurality of recessed portions (102) have a depth D and a hole diameter S; and

$$20 \text{ nm} \leq D \leq 400 \text{ nm and } D/S \leq 1.1$$

are satisfied.

14. The cell culture vessel according to claim 13, wherein a recess/protrusion pattern height difference HD of the periodic recess/protrusion pattern satisfies

$$20 \text{ nm} \leq HD \leq 400 \text{ nm.}$$

15. The cell culture vessel according to claims 13 or 14, wherein the culture surface satisfies at least any one of:

contiguity between a highest surface and a side surface through a curved surface portion; and
contiguity between a lowest surface and a side surface through the curved surface portion.

16. The cell culture vessel according to any one of claims 13 to 15, wherein the plurality of protruding portions or the plurality of recessed portions each have one of a cylindrical shape or a truncated conical shape.

## FIG. 1

## FIG. 2A

# FIG. 2B

## FIG. 3A

## FIG. 3B

## FIG. 3C

FIG. 3D

FIG. 3E

*FIG. 4*

```
        ┌─────────────┐
        │    START    │
        └──────┬──────┘
               │
               ▼
   ┌───────────────────────┐
   │     CULTURE STEP      │ ～S310
   └───────────┬───────────┘
               │
               ▼
   ┌───────────────────────┐
   │    DETACHMENT STEP    │ ～S320
   └───────────┬───────────┘
               │
               ▼
   ┌───────────────────────┐
   │ COLLECT DETACHED CELLS│ ～S330
   └───────────┬───────────┘
               │
               ▼
        ┌─────────────┐
        │     END     │
        └─────────────┘
```

# FIG. 5

# FIG. 6A

## FIG. 6B

# FIG. 7A

# FIG. 7B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005168494 A

- JP 2019037151 A